# EUROPEAN PATENT APPLICATION

(11) **EP 4 122 485 A1**
(43) Date of publication of application: **25.01.2023**
(21) Application number: 21382657.1
(22) Date of filing: 20.07.2021
(51) Int. Cl.: A61K 38/17, A61P 35/00, A61K 31/495

(54) **CONNEXIN 43 FOR USE IN THE TREATMENT OF RETINOBLASTOMA PROTEIN POSITIVE TUMOURS**

(71) Applicant: Fundación Profesor Novoa Santos, 15006 A Coruña (ES); Servizo Galego de Saude, 15703 Santiago de Compostela (ES)
(72) Inventor: MAYÁN SANTOS, María Dolores, 15006 A Coruña (ES); CARPINTERO FERNÁNDEZ, Paula, 15006 A Coruña (ES); GARCÍA YUSTE, Alejandro, 15006 A Coruña (ES); ACEA NEBRIL, Benigno, 15001 A Coruña (ES); FONSECA CAPDEVILA, Eduardo, 15001 A Coruña (ES)
(74) Representative: Hoffmann Eitle

(57) **Abstract**

The present invention to connexin 43 (Cx43) for use in the treatment of a type of cancer characterized by the presence of functional retinoblastoma gene (RB), or by the presence of functional RB and also by hyperactivity of CDK4 and CDK6, preferably in combination with a CDK4/6 inhibitor.

## Description

### FIELD OF THE INVENTION

The present invention refers to the medical field. Particularly, the present invention refers to connexin 43 (Cx43) for use in the treatment of a type of cancer characterized by the presence of functional retinoblastoma gene (RB), or by the presence of functional RB and also by hyperactivity of CDK4 and CDK6, preferably in combination with a CDK4/6 inhibitor.

### STATE OF THE ART

A key characteristic of the majority of cancer cells is the deregulation of cell cycle checkpoint proteins such as the cyclin-dependent kinases (CDKs) CDK4 and CDK6 leading to uncontrolled cell proliferation. The cell-cycle kinases CDK4 and CDK6 phosphorylate retinoblastoma protein (pRb) family members and regulate progression through G1 and entry into the DNA synthesis (S) phase. CDK4 and CDK6 bind to D-type cyclins D1, D2, and D3, leading to CDK4/6 activation and pRb phosphorylation. CDK-dependent phosphorylation of pRb results in its inactivation and allows for the transcriptional induction of genes required for cell cycle progression. Most tumor types display functional upregulation of these kinases through overexpression (such as amplification or translocation) of D-type cyclins or display inactivation of the inhibitors of the cyclin D-CDK4/6, which include the INK4 proteins, composed of 4 inhibitors: p16INK4a, p15INK4b, p18INK4c, and p19INK4d. These inhibitors specifically inactivate CDK4 and CDK6 by inducing structural changes that avoid binding and activation by D-type cyclins. p16INK4a, p15INK4b, and p18INK4c are found inactivated in many tumors by deletion, mutation, or gene promoter activity regulation.

Molecular changes at CDKs levels have been reported in various cancer types making them an attractive potential target for new treatments. CDK inhibitors, in particular CDK4/6 inhibitors (such as Abemaciclib, Palbociclib and Ribociclib) induce cell-cycle arrest and subsequent cellular senescence in human cancer cell lines. Mechanistically, CKD4/6 inhibitors inhibit CDK4/6 activity and the phosphorylation of Rb, stabilizing the Rb-E2F inhibitory complex and inhibiting therefore the activity of the E2F transcription factor family that regulate cell cycle progression and apoptosis. Particularly, Palbociclib (PD-0332991), has shown effectiveness specially in advanced HR+/HER2- breast cancer, improving the progression-free survival from 18 to 27 months and in advanced HR+ breast cancer, Palbociblib showed beneficial effects compared to hormone therapy, letrozole or fulvestrant when using alone. Although their effectiveness has been proven mainly in breast cancer, there are undergoing several clinical trials in a variety of other (Rb positive) cancer types such as squamous cell lung cancer (NCT02785939), pancreatic neuroendocrine tumours (NCT02806648) and oligodendroglioma and oligoastrocytoma (NCT02530320).

As previously mentioned, targeting cell cycle checkpoints, such as CDK4 and CDK6 in Rb positive cancer cells and/or with hyperactivity of CDK4 and CDK6, with specific CDK4/6 inhibitors improved progression-free survival in breast cancer patients and this effect is due to the acquisition of a new phenotype by cancer cells called cellular senescence.

Cellular senescence is as a state by which cells lose their proliferative capacity despite, they are metabolically active. Senescent cells also secrete a complex of pro-inflammatory molecules known as senescence-associated secretory phenotype (SASP). The SASP includes the secretion of cytokines, enzymes, chemokines and macrophage inflammatory proteins that causes inflammation, and, in some cases, it may be pivotal for the clearance of senescent cells by phagocytosis. Although most cell types follow the senescence program and its beneficial effects; cancer cells tent to avoid cellular senescence resulting in uncontrolled cellular proliferation and tumorigenesis. For this reason, the induction of senescence, together with the consequent elimination of these cells from the tissues are already considered a new anti-cancer strategy.

Thus, CDK4/6 inhibitors are considered a highly selective class of new generation therapeutics that bind to the CDK4 and CDK6 ATP-binding pocket, leading to the inactivation of CDK4/6-Cyclin D complexes with the subsequent inhibition of Rb phosphorylation and induction of G1 phase arrest and cellular senescence in target cancer cells. Despite these findings, specific molecular mechanisms by which CDK4/6 inhibitors have an anti-tumour activity and induce a stable cell cycle arrest in cancer cells are still under study. This lack of information does not allow patient stratification or to develop therapeutic strategies to avoid drug resistance in order to increase efficacy and the progression free survival of advanced cancer patients.

Consequently, there is an unmet medical need of finding new therapeutic strategies to reduce CDK4/6 inhibitors innate and acquired resistance and to improve their efficacy in patients with Rb positive tumours or in tumours with overexpression or overactivity of CDK4 and CDK6, in order to ameliorate cancer relapse.

The present invention is focused on solving this problem by providing a new strategy for reducing CDK4/6 and Rb activity and modifying the activity of other cell cycle regulators in order to increase CDK4/6 inhibitors efficacy and to avoid drug resistance, improving their efficacy in patients with a metastatic disease ameliorating cancer relapse. The combination of the present disclosure may also be useful in the treatment of tumours with genetic mutations or changes that activate CDK4 and CDK6 activity, amplifications, overactivity or overexpression of CDK4 or CDK6, tumours harbouring p16INK4a, p15INK4b, and p18INK4c inactivation or tumours over-expressing cyclin partners of the cyclin dependent kinases (D1, D2, and D3). This disclosure may also be useful in the treatment of Rb-negative tumours.

### DESCRIPTION OF THE INVENTION

### Brief description of the invention

As explained above, the present invention provides a new strategy aimed at reducing CDK4/6 inhibitors innate and acquired resistance by improving their efficacy in patients with Rb positive tumours, thus ameliorating cancer relapse.

Particularly, according to the results shown below, the inventors of the present invention demonstrate that using Cx43, in combination with CDK4/6 inhibitors, significantly increases CDK4/6 inhibitors efficacy. Moreover, the combination of Cx43, CDK4/6 inhibitors and senolytic drugs, such as Navitoclax, has been assayed and this combination resulted in the best strategy to reduce the proliferation of Rb positive breast cancer cells.

Specifically, the results disclosed in the present invention shows that the restoration of Cx43 in Rb positive cancer cells induces proliferation arrest (see **Example 1** and **Figure 1****),** Cx43 overexpression induces a senescent-like phenotype in Rb positive cancer cells (see **Example 2** and **Figure 2****),** Cx43 overexpression increases the efficacy of CDK4/6 inhibitors (see **Example** 3 and **Figure 3****),** the combination of Cx43 and CDK4/6 inhibitors enhances the effect of the senolytic drug Navitocax (see **Example 4** and **Figure 4****),** Cx43 increases the efficacy of CDK4/6 inhibitors in other tumours types (see **Example 5** and **Figure 5****)** and the delivery of Cx43 using vesicles (sEVs) has anti-tumour activity and improve CDK4/6i efficacy (see **Example 6** and **Figure 6****).**

So, the first embodiment of the present invention refers to Cx43 for use in the treatment of cancer, wherein the cancer is characterized by the presence of functional retinoblastoma gene (RB) and/or by hyperactivity of CDK4/6. Kindly note that cancer types characterized by the presence of functional retinoblastoma gene (RB) (which means that RB is active and pRb protein levels are not significantly depleted) are well-established in the prior art, such as it is indicated for instance in these documents: [Goel S, DeCristo MJ, McAllister SS, Zhao JJ. CDK4/6 Inhibition in Cancer: Beyond Cell Cycle Arrest. Trends Cell Biol. 2018;28(11):911-25*], [*Condorelli R, Spring L, O'Shaughnessy J, Lacroix L, Bailleux C, Scott V, et al. Polyclonal RB1 mutations and acquired resistance to CDK 4/6 inhibitors in patients with metastatic breast cancer. Ann Oncol. 2018;29(3):640-5*]* or [Pandey K, An HJ, Kim SK, Lee SA, Kim S, Lim SM, et al. Molecular mechanisms of resistance to CDK4/6 inhibitors in breast cancer: A review. Int J Cancer. 2019;145(5):1179-88*].*

In a preferred embodiment of the present invention Cx43 is used before, after or simultaneously to a treatment with a CDK4/6 inhibitor.

In a preferred embodiment of the present invention the CDK4/6 inhibitor is selected from: Abemaciclib, Palbociclib and/or Ribociclib.

In a preferred embodiment of the present invention, the cancer which is characterized by the presence of functional retinoblastoma gene (RB) is selected from the list comprising: breast cancer, melanoma, lung cancer, colon, squamous cell lung cancer, pancreatic neuroendocrine tumour, oligodendroglioma and oligoastrocytoma among others.

In a preferred embodiment of the present invention, the cancer is characterized by p16INK4a, p15INK4b, and p18INK4c inactivation or tumours over-expressing cyclin partners of the cyclin dependent kinases (D1, D2, and D3). This disclosure may also be useful in the treatment of RB-negative tumours and selected from the list comprising: breast cancer, melanoma, lung cancer, colon, squamous cell lung cancer, non-small cell lung cancer, cervical cancer, stomach cancer, endometrium cancer, ovarian cancer, urinary track, pancreatic neuroendocrine tumour, oligodendroglioma and oligoastrocytoma, hematopoietic tumours of lymphoid lineage.

The second embodiment of the present invention refers to a combination drug product comprising Cx43 and a CDK4/6 inhibitor.

In a preferred embodiment of the present invention, the combination drug product comprises Cx43 protein or Cx43 mRNA.

In a preferred embodiment of the present invention, the CDK4/6 inhibitor included in the combination drug product is selected from the group comprising: Abemaciclib, Palbociclib and/or Ribociclib.

In a preferred embodiment of the present invention, the combination drug product further comprises a senolytic agent, preferably navitoclax.

The third embodiment of the present invention refers to a pharmaceutical composition comprising the combination drug product of the invention and, optionally, pharmaceutically acceptable excipients or carriers.

The fourth embodiment of the present invention refers to the pharmaceutical composition of the invention for use in the treatment of cancer wherein the cancer is characterized by the presence of functional retinoblastoma gene (RB).

In a preferred embodiment of the present invention, Cx43 is administered by using a delivery vehicle.

In a preferred embodiment of the present invention, the delivery vehicle is a nanoparticle, a vesicle, an extracellular vesicle or an expression vector. Particularly, any of the extracellular vesicles known in the prior art may be used for this purpose [Guillaume van Niel, et al., 2018. Shedding light on the cell biology of extracellular vesicles. Nature Reviews Molecular Cell Biology volume 19, pages 213-228(2018*)] [*Oscar P. B. Wiklander et al., 2019. Advances in therapeutic applications of extracellular vesicles. Science Translational Medicine. 15 May 2019. Vol. 11, Issue 492, eaav8521. DOI: 10.1126/scitranslmed.aav8521*].*

In a preferred embodiment of the present invention, the expression vector encodes Cx43 mRNA which in turn is translated into Cx43 protein.

The last embodiment of the present invention refers to a method for treating cancer, wherein the cancer is characterized by the presence of functional retinoblastoma gene (RB) and/or by the hyperactivity of CDK4/6, which comprises the administration of a therapeutically effective amount of Cx43, preferably in combination with a CDK4/6 inhibitor, and most preferably, also in combination with a senolytic agent (as defined above), or a pharmaceutical composition comprising thereof.

In the context of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of' means including, and it is limited to, whatever follows the phrase "consisting of'. Thus, the phrase "consisting of' indicates that the listed elements are required or mandatory, and that no other elements may be present.
- "Pharmaceutically acceptable excipient or carrier" refers to an excipient that may optionally be included with the cell suspension of the invention and that causes no significant adverse toxicological effects to the patient.
- By "therapeutically effective dose or amount" of a composition comprising the cell suspension of the invention is intended an amount that, when administered as described herein, brings about a positive therapeutic response in a subject having cancer. The exact amount required will vary from subject to subject, depending on the age, and general condition of the subject, the severity of the condition being treated, mode of administration, and the like. An appropriate "effective" amount in any individual case may be determined by one of ordinary skill in the art using routine experimentation, based upon the information provided herein.
- By "cancer types characterized by the presence of functional retinoblastoma gene (RB)" is understood any type of cancer wherein RB is active and pRb protein levels are not significantly depleted, or RB positive tumors. This type of cancer pertains to the common general knowledge and is cited in several documents like: [Knudsen ES, Wang JY (February 2010). "Targeting the RB-pathway in cancer therapy". Clinical Cancer Research. 16 (4): 1094 9. doi:10.1158/1078-0432.CCR-09-0787. PMC 2822892. PMID 20145169*], [*Goel S, DeCristo MJ, McAllister SS, Zhao JJ. CDK4/6 Inhibition in Cancer: Beyond Cell Cycle Arrest. Trends Cell Biol. 2018;28(11):911-25*], [*Condorelli R, Spring L, O'Shaughnessy J, Lacroix L, Bailleux C, Scott V, et al. Polyclonal RBI mutations and acquired resistance to CDK 4/6 inhibitors in patients with metastatic breast cancer. Ann Oncol. 2018;29(3):640-5*]* or [Pandey K, An HJ, Kim SK, Lee SA, Kim S, Lim SM, et al. Molecular mechanisms of resistance to CDK4/6 inhibitors in breast cancer: A review. Int J Cancer. 2019; 145(5): 1179-88*],*

### Description of the figures

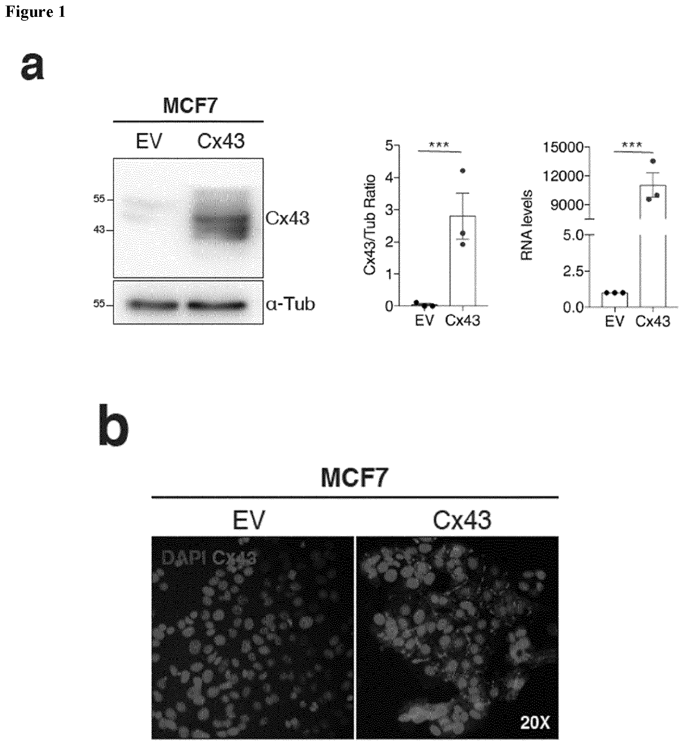

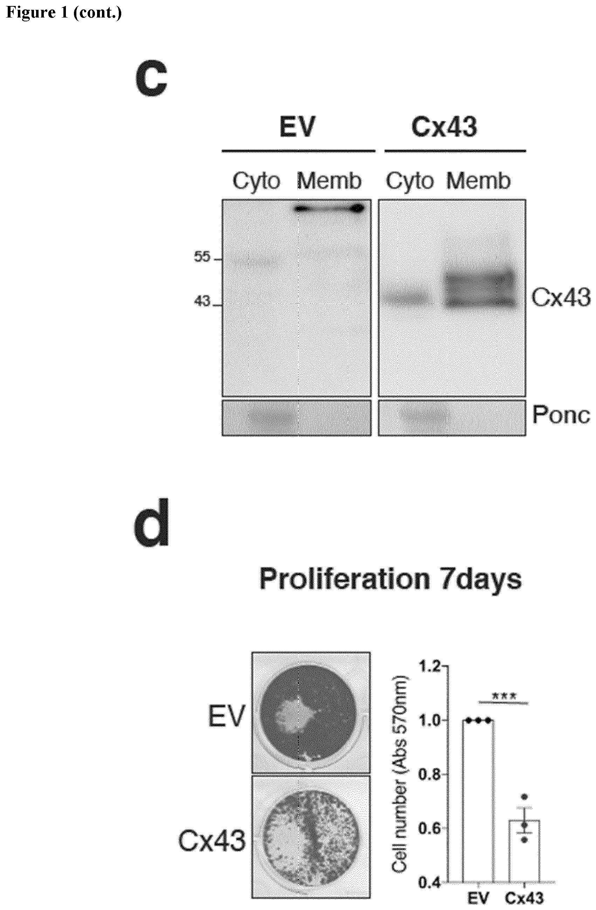

**Figure 1****. Cx43 in Rb positive cancer cells induces proliferation arrest. (a)** Rb positive breast cancer cells (MCF7) were infected with a vector to overexpress Cx43. Cx43 protein and mRNA levels were tested by Western-blot (protein) and qPCR (mRNA) comparing the vector control (empty vector; EV) to MCF7 cells overexpressing Cx43 (Cx43). Tubulin was used as loading control. **(b)** Immunofluorescence showing Cx43 overexpression and localization in MCF7 cells. **(c)** Western-blot demonstrating that Cx43 is localized at the plasma membrane in MCF7 cells after Cx43 overexpression compared to the EV. Ponceau was used as loading control. **(d)** Colony formation assay to demonstrate that Cx43 overexpression induces proliferation arrest in MCF7 cells after 7 days in culture. Data represent the mean ± SEM of 3 independent experiments. Two-tailed Student's t-test was used to calculate the significance represented as followed: *p<0.05; **p<0.01; ***p<0.01.

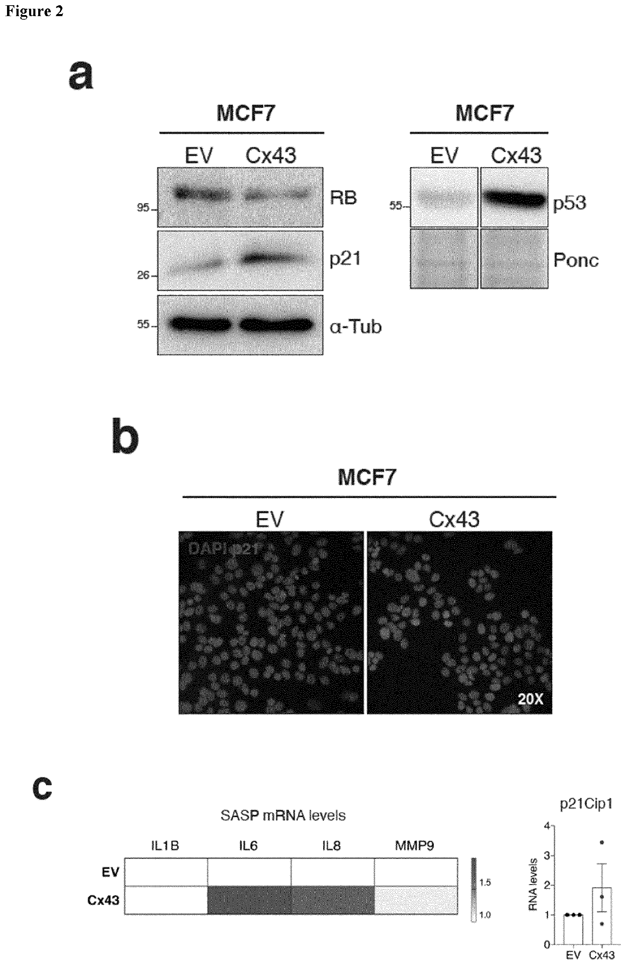

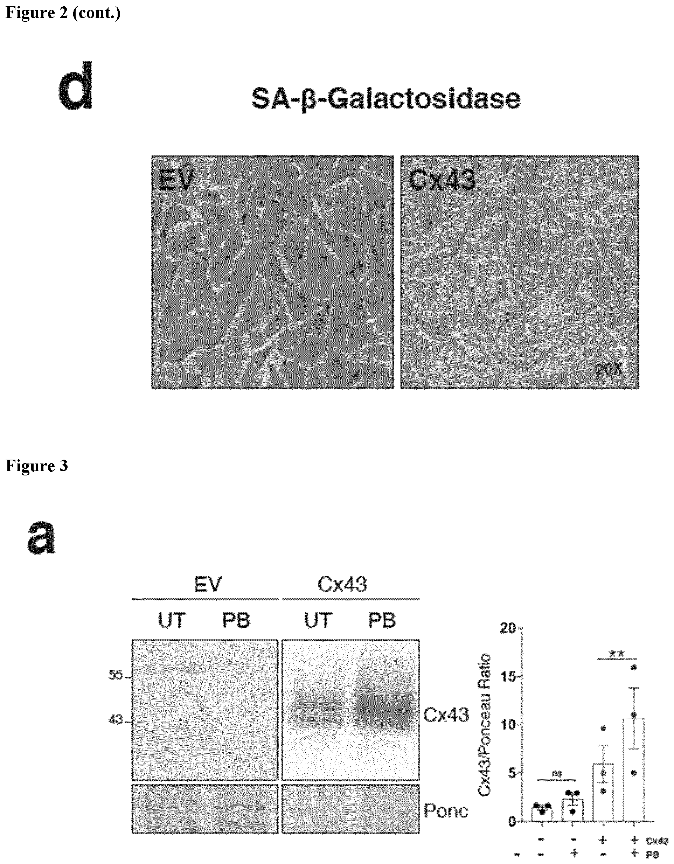

**Figure 2****. Cx43 induces a senescent-like phenotype in Rb positive cancer cells. (a)** Western blot shows the upregulation of the senescence markers p53 and p21^{CIP1} and a reduction in total RB levels when cells overexpress Cx43. Tubulin and ponceau were used as loading controls. **(b)** Representative images of p21^{CIP1} (red) of MCF7 after Cx43 overexpression. **(c)** Heat map of SASP gene expression in MCF7 cells expressing the control vector or Cx43. The mean of 3 independent experiments is shown. The graph represents the mRNA levels of p21^{CIP1} in MCF7 upon Cx43 overexpression. **(d)** SA-β-galactosidase activity to detect senescent cells (blue) in MCF7 expressing Cx43 shown by light microscopy images. The data represent the mean ± SEM of 2-3 independent experiments. Two-tailed Student's t-test was used to calculate the significance represented as followed: *p<0.05; **p<0.01; ***p<0.01.

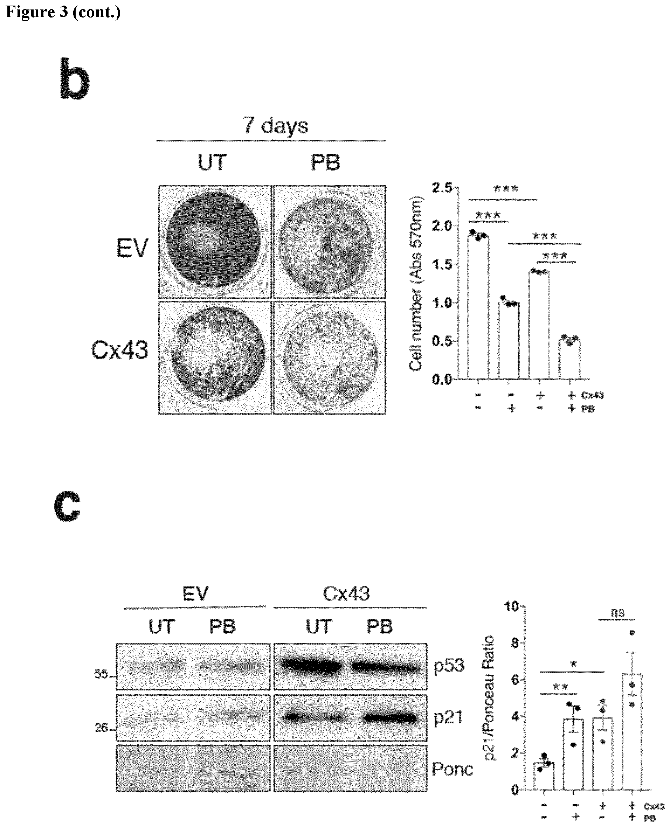

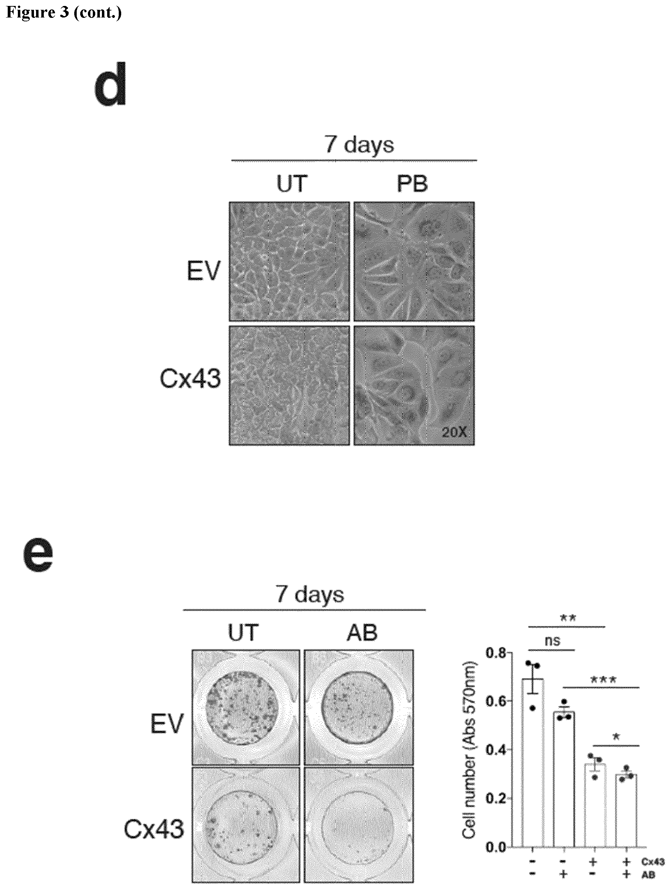

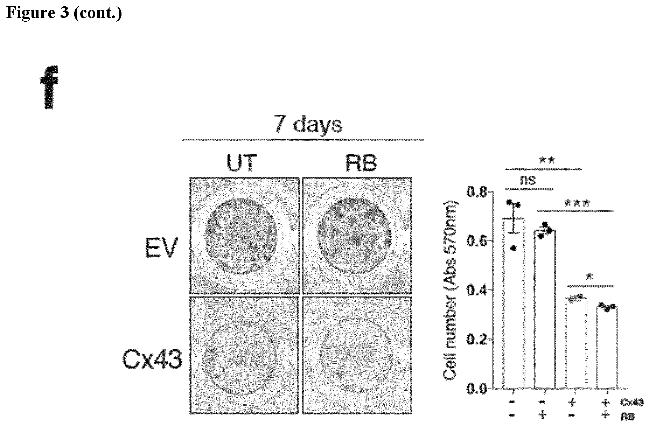

**Figure 3****. Cx43 increases the efficacy of CDK4/6 inhibitors. (a)** Western blot for Cx43 protein levels after 7 days of treatment with 500nM Palbociclib (PB) compared to the untreated control (UT). The graph represents the quantification of 3 independent experiments. Ponceau was used as loading control. **(b)** Colony formation capacity of MCF7 cells expressing the vector control (EV) or Cx43 overexpression (Cx43) and treated with 500nM Palbociclib for 7 days. **(c)** p21^{CIP1} protein levels in MCF7 after Cx43 overexpression and 7 days Palbo treatment. Quantification is shown on the right. Ponceau was used as loading control. **(d)** Representative images of SA-β-galactosidase activity in MCF7 expressing EV or Cx43 and treated with Palbo for 7 days. Images were taken using a light microscope. **(e)** Crystal violet staining was used to check the colony formation capacity of MCF7 expressing EV or Cx43 treated with 500nM Abemaciclib (AB) for 7 days. Quantification is shown on the right **(f)** Colony formation capacity of MCF7 cells overexpressing Cx43 after 7 days treatment with the CDK4/6 inhibitor Ribociclib (RB). Quantification is shown on the right. The data represent the mean ± SEM of 2-3 independent experiments. Two-tailed Student's t-test was used to calculate the significance represented as followed: *p<0.05; **p<0.01; ***p<0.01.

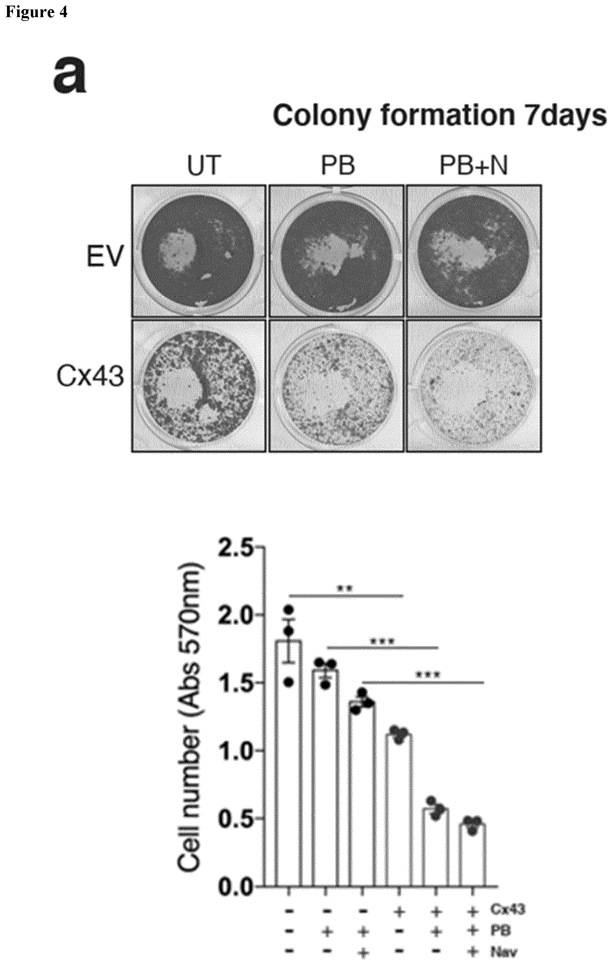

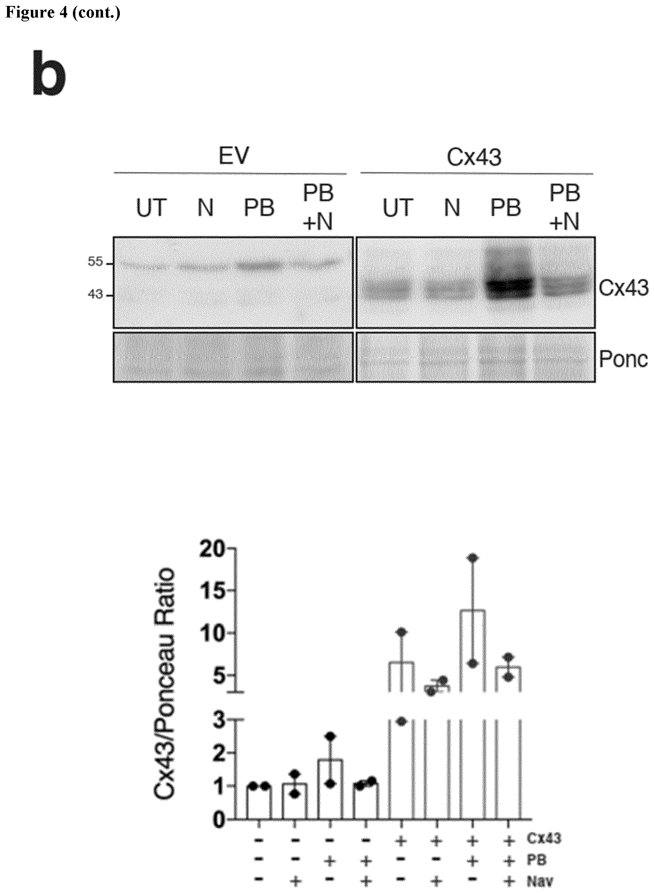

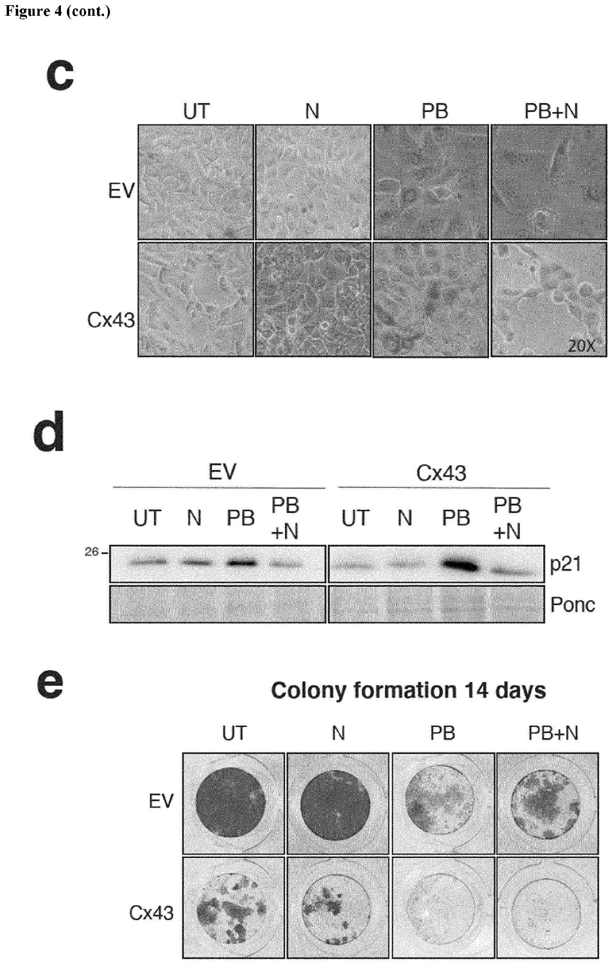

**Figure 4****. The combination of Cx43 and CDK4/6 inhibitors enhances the effect of the senolytic drug Navitocax. (a)** Colony formation capacity of MCF7 cells expressing the vector control (EV) or Cx43 (Cx43) and treated with 500nM Palbociclib for 7 days and 24 hours with 5µM Navitoclax (N). **(b)** Cx43 protein levels in MCF7 after Cx43 overexpression and 7 days Palbo treatment and 24 hours with 5µM Navitoclax (N). Quantifications are shown on the right. Ponceau was used as loading control. **(c)** Representative images of SA-β-galactosidase activity in MCF7 expressing EV or Cx43 and treated with Palbo for 7 days and 24 hours with 5µM Navitoclax (N). Images were taken using a light microscope. The higher reduction in the number of tumour senescent cells occurred under the triple combination of Cx43 with Palbociclib and Navitoclax (PB+N) **(̵d)** p21^{CIP1} protein levels in MCF7 after Cx43 overexpression and 7 days Palbo treatment and 24 hours with 3.5µM Navitoclax (N). Ponceau was used as loading control. **(e)** Colony formation assay of MCF7 cells expressing the vector control (EV) or Cx43 overexpression (Cx43) and treated with 500nM Palbociclib for 14 days and 3.5µM Navitoclax (N) for 7 days. The data represent the mean ± SEM of 2-3 independent experiments. Two-tailed Student's t-test was used to calculate the significance represented as followed: *p<0.05; **p<0.01; ***p<0.01.

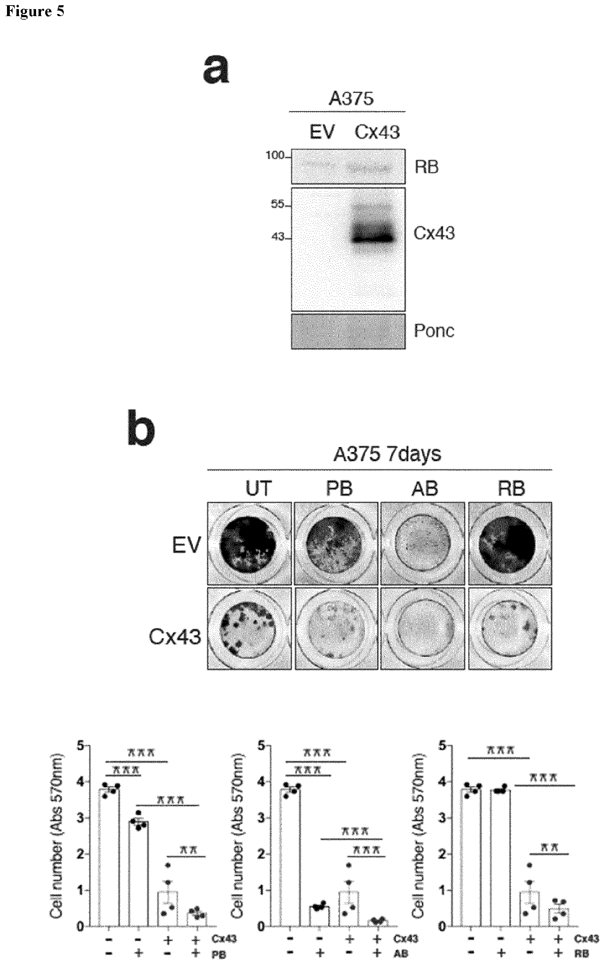

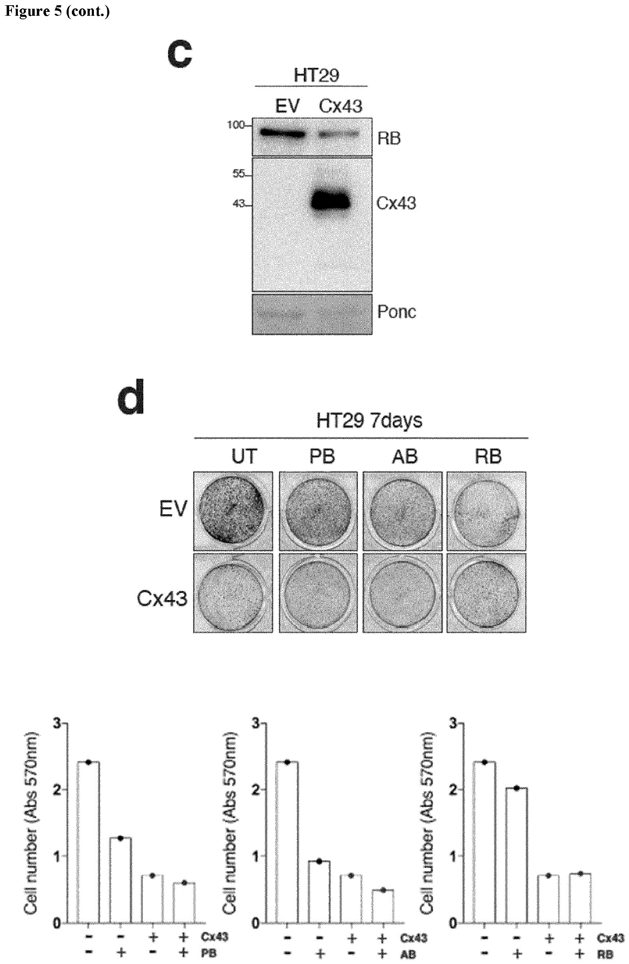

**Figure 5****. Cx43 increases the efficacy of CDK4/6 inhibitors in other tumours types. (a)** Western-blot of human A375 melanoma cells after transfection with a vector to overexpress Cx43. Rb and Cx43 protein levels are shown. Ponceau was used as loading control. EV (vector control); Cx43 (cells overexpressing Cx43). **(b)** Colony formation assay of A375 cell line after 7 days treatment with 250nM Palbociclib (PB) and 500nM Abemaciclib (AB) and Ribociclib (RB). **(c)** Western-blot of HT29 colon adenocarcinoma cells after transfection. Rb and Cx43 protein levels are shown. Ponceau was used as loading control. EV (vector control); Cx43 (cells overexpressing Cx43). **(d)** Proliferation of HT29 colon cells after 7 days treatment with 250nM Palbociclib (PB) and 500nM Abemaciclib (AB) and Ribociclib (RB). The data represent the mean ± SEM of 2-3 independent experiments. Two-tailed Student's t-test was used to calculate the significance represented as followed: *p<0.05; **p<0.01; ***p<0.01.

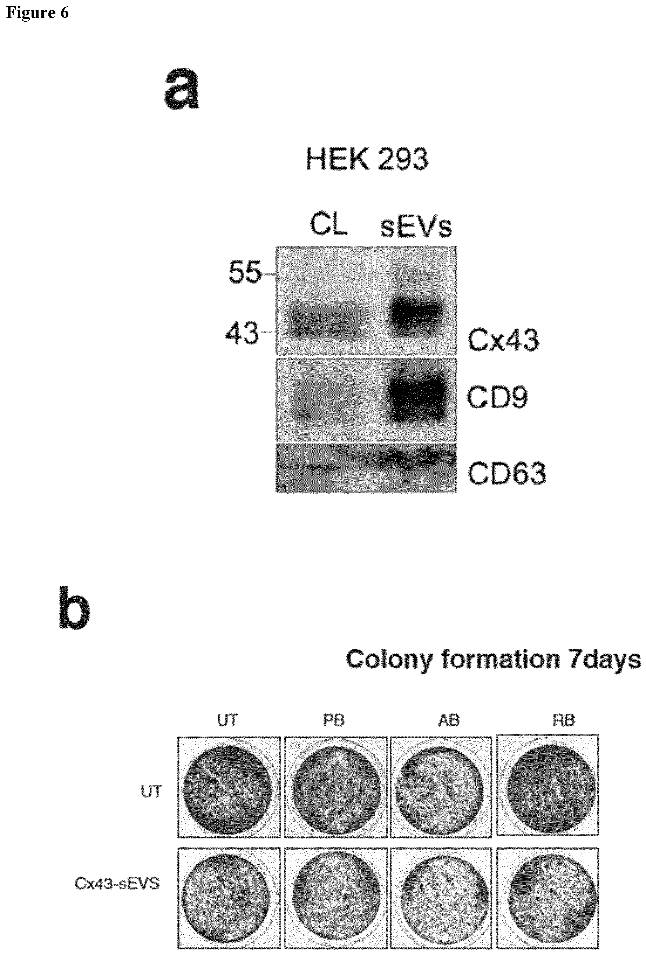

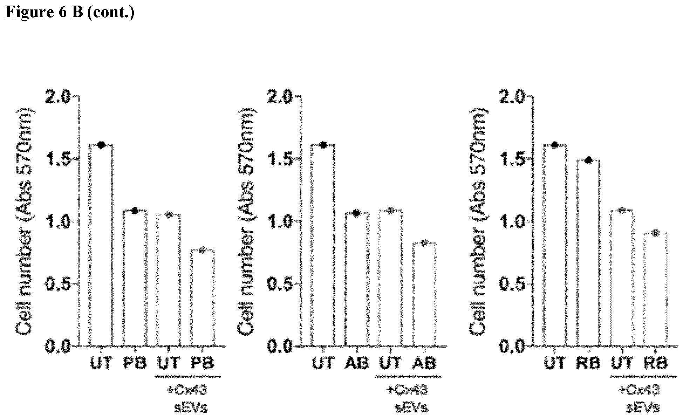

**Figure 6****. The delivery of Cx43 using Vesicles (sEVs) has a potent anti-tumour activity and improve CDK4/6i efficacy. (a)** Western-blot showing that Cx43 is present in high levels in the sEVs isolated from HEK-293, used as delivery vehicle to increase Cx43 in tumour cells. CD9 and CD63 were used as markers to confirm the presence of sEVs compared to cell lysate (CL). **(b)** Colony formation assay of MCF7 breast cancer cells treated with 500nM of Palbociclib, 1µM of Abemaciclib and 1µM of Ribociclib alone or in combination of sEVs positive for Cx43 isolated from HEK293 for 7 days. The presence of sEVs enriched in Cx43 decreases the capacity to form colonies and increases the efficacy of treatment. Treatments and sEVs were refreshed every 48 h. UT (untreated), sEVs (sEVs derived from HEK-293).

### Detailed description of the invention

The present invention is illustrated by means of the examples set below without the intention of limiting its scope of protection.

### Example 1. Materials and methods

### Example 1.1. Cell culture

Cell lines MCF7 (breast cancer) and A375 (melanoma) were cultured in Dulbecco's modified Eagle's medium (DMEM) (Lonza) supplemented with 10 % fetal bovine serum (FBS) (Gibco, Thermo Fisher Scientific) and 100 U/mL penicillin and 100 µg/ml streptomycin (Gibco, Thermo Fisher Scientific). HT29 (colon adenocarcinoma) was cultured in McCoy'S 5A medium (Sigma-Aldrich) supplemented with 10 % FBS and 100 U/mL penicillin and 100 µg/ml streptomycin. Cells were maintained at 37 °C in 5 % CO₂ humidified incubator (SANYO CO²). The medium was changed every 2 days.

### Example 1.2. Retroviral infection

Retroviral infection was used to overexpress Cx43 in MCF7 cells. Retroviral particles were generated by transfecting pLPCX-Cx43 and pLPCX-EV plasmids, kindly donated by Dr. Trond Aasen, and retroviral helper plasmids (vsvg and gag-pol) with Polyethylenimine (PEI) in HEK293T packaging cells for 48h. The supernatant was then filtered with 0.45µm filters and applied to MCF7 cells in the presence of 4µg/ml polybrene (hexadimethrine bromide; Sigma-Aldrich) following 3 rounds of infection. Cells were subsequently selected with the appropriate antibiotic resistance using 0.5µg/ml puromycin (Sigma).

### Example 1.3. Cell Transfection

Cell lines (A375 and HT29) were transfected by electroporation to overexpress Cx43. The Amaxa^{®} Cell Line Nucleofector^{®} Kit V (Lonza) was used to transfect cells in a Cell Line Nucleofector^{™} Device (Lonza). A million cells were harvested and resuspended in 100 µL of Nucleofector^{®} solution. 3 µg of the corresponding plasmid was added, and the mixture was transferred into an electroporation cuvette using the U-28 program. Cell lines were transfected with pIRESpuro2 plasmid construct (Clontech) containing the human Cx43 sequence, kindly donated by Arantxa Tabernero (Institute of Neuroscience of Castilla y León, University of Salamanca, Spain). The transfected cells were seeded in culture medium and 24 hours (h) post-transfection the medium was replaced with complete medium containing puromycin dihydrochloride (Tocris, Bioscience) at different concentrations depending of the cell line (0,5 µg/mL to 2 µg/mL).

### Example 1.4. CDK4/6 inhibitors treatment

Cells were treated with 250nM of Palbociclib (PD0332991) (APExBIO), 500nM Abemaciclib (LY2835219) (Selleckchem) or 500nM Ribociclib (LEE011) (Selleckchem) for 7 days. All treatments were done using DMEM supplemented with 10% FBS and 100 U/mL penicillin and 100 µg/ml streptomycin. Media was replaced every 2 days.

### Example 1.5. Immunofluorescence

Cells were grown onto coverslips and fixed with 4% paraformaldehyde (PFA) for 10 min at room temperature (RT). Cells were then washed twice with PBS and permeabilized by incubation with 0.2% Triton X-100 (Sigma) in PBS for 10 min at RT. After a PBS wash, cells were blocked 30 min at RT using 1% BSA in PBS supplemented with 0.1% Tween-20 (PBST). Primary antibodies were diluted in 1% BSA-PBST and incubated overnight (anti- Cx43 (#C6219), Sigma- Aldrich 1: 1000; anti-Tubulin (T9026). Cells were then washed with PBS and incubated with secondary antibodies for 1 h at RT. HRP-conjugated secondary antibodies used were anti-mouse (NA-931, Sigma-Aldrich) and anti-rabbit (A6154, Sigma-Aldrich).

### Example 1.6. Colony formation assay

Colony formation assays were performed plating 5 x 10³ to 5 x 10⁴ cells per well onto 6 - 12 well plates and grown for 7 - 15 days. Medium was replaced every 48 h. The cells were washed with warm Saline Solution and fixed with cold 4 % PFA for 15 min. Cells were washed with PBS and stained with 0.05 % of crystal violet (Sigma-Aldrich) for 15 min. After staining, the cells were washed with distilled water and dried at room temperature. The colonies were quantified by diluting crystal violet in 30 % acetic acid and 100 µL of the solution was collected to measure the absorbance at 570 nm using a NanoQuant microplate reader Infinite M200 (TECAN).

### Example 1.7. β-gatactosidase staining

Cells were washed with PBS and fixed with 4% paraformaldehyde for 15mins at room temperature. Cells were washed a second time with PBS and incubated with 5-bromo-4-chloro-3-indolyl-beta-D-galacto-pyranoside (X-gal) solution for O/N at 37°C (Senescence Cells Histochemical Staining Kit Ref CS0030-1KT; Sigma). Cells were imaged using a light microscope at 20X magnification and single representative images of each well were taken.

### Example 1.8. RNA extraction and reverse transcription polymerase chain reaction (RT-qPCR)

Total RNA was isolated from cells using TRI Reagent^{®} RT (Vitro Bio) according to manufacturer's protocol. 200 µL of chloroform were added to the tubes and samples were incubated for 10 min at 4 °C and then centrifugated at 14,000 g for 15 min at 4 °C. After centrifugation, the aqueous phase (upper) are obtained and transferred to a new tube with 500 µL of isopropanol and samples were vortexed 15 sec. The tubes centrifugated at 14,000 g for 15 min at 4 °C. Supernatant was discarded, and the RNA pellets were washed with 1 mL of 70 % ethanol and centrifugated for 5 min at 7,000 g at 4 °C. The supernatants were discarded, and RNA pellets were air dry. RNA was treated with DNase I, RNase free (Thermo Fisher Scientific) following manufacturer's protocol. RNA samples were quantified in a Nanodrop^{®} ND-1000 (Thermo Fisher Scientific). 1 µg of RNA was used to synthesize complementary DNA (cDNA) with the Superscript^{™} IV VILO^{™} Master Mix (Invitrogen, Thermo Fisher Scientific) following manufacturer's protocol. cDNA samples are quantified in a Nanodrop^{®} ND-1000 (Thermo Fisher Scientific) and resuspended in DNase/RNase/Protease-free water (Sigma-Aldrich).

### Example 1.9. Real time quantitative PCR (RT-qPCR)

5 µL of cDNA (1µg) were mixed with 0.5 µL of primer mix (10 µM each primer), 10 µL of Applied Biosystems^{™} PowerUP^{™} SYBR^{™} Green Master Mix (Applied Biosystems, Thermo Fisher Scientific) and complete with dH₂O until 20 µL per well on LightCycler^{®} 480 System (Roche). The program consisted on a first denaturing cycle of 10 min at 95 °C followed by 30 - 55 amplification cycles for 10 seconds (sec) at 95 °C, 30 sec at 60 °C for annealing and 12 sec at 72 °C for extension. The primers used are listed below:

| **Gene Name** | **Forward Sequence (5'-3')** | **Reverse Sequence (5'-3')** |
|---|---|---|
| **CDKN1A (p21)** | GACTCTCAGGGTCGAAAACG (SEQ ID NO: 1) | GCCAGGGTATGTACATGAGGA (SEQ ID NO: 2) |
| ***GJA1* (Cx43)** | ACATGGGTGACTGGAGCGCC (SEQ ID NO: 3) | ATGATCTGCAGGACCCAGAA (SEQ ID NO: 4) |
| ***IL1β*** | CGAATCTCCGACCACCACTAC (SEQ ID NO: 5) | TCCATGGCCACAACAACTGA (SEQ ID NO: 6) |
| ***IL6*** | TGTAGCCGCCCCACACA (SEQ ID NO: 7) | GGATGTACCGAATTTGTTTGTA (SEQ ID NO: 8) |
| ***IL8*** | TTGGCAGCCTTCCTGATTTC (SEQ ID NO: 9) | TCTTTAGCACTCCTTGGCAAAAC (SEQ ID NO: 10) |
| ***MMP9*** | TTCATCTTCCAAGGCCAATC (SEQ ID NO: 11) | GGGCTGAACCTGGTAGACAG (SEQ ID NO: 12) |
| ***HPRT1*** | TTGAGTTTGGAAACATCTGGAG (SEQ ID NO: 13) | GCCCAAAGGGAACTGATAGTC (SEQ ID NO: 14) |

### Example 1.10. Small Extracellular vesicles (sEVs) isolation and treatment

Cells were seeded onto 162 cm² culture flasks (Corning, Sigma-Aldrich) until confluence (80 % - 90 %). Cells were washed three times with PBS and cultured for 48 h in DMEM 0 % FBS. Supernatants were collected and centrifuged at 1800 rpm for 5 min. Pellet was discarded and supernatants were filtered through a 0.22 µm filter and then the filtered supernatants were centrifuged at 100,000 g for 90 min at 4 °C (Optimal-90K ultracentrifuge) using 70 Ti rotor (Beckman Coulter). Supernatants were discarded and pellet-containing sEVs were washed with PBS and centrifuged at 100,000 g for 90 min at 4 °C. The pellet with sEVs was resuspended in culture medium. Target cells were treated with sEVs in the presence/absence of CDK4/6 inhibitors for 48 h. 3 rounds of treatment were performed.

### Example 2. Results

### Example 2.1. Cx43 in Rb positive cancer cells induces proliferation arrest

Here we used the human breast cancer cell line MCF7 to overexpress Cx43. Cx43 was overexpressed by using a retroviral vector and protein overexpression was confirmed using wester-blot (protein) and RT-PCR (mRNA levels) **(****Figure 1a**). Since connexins activity and/or function depend on their localization, we checked Cx43 localization in MCF7 cells overexpressing Cx43 by using immunofluorescence **(****Figure 1b**) and western-blot **(****Figure 1c**). As shown in **Figure 1b**, Cx43 was mainly localized at the plasma membrane (in red) compared to MCF7 cells expressing the vector control (EV) that express very low levels of Cx43. Besides, western-blot of cytoplasm (soluble) and membrane (insoluble) cellular fractions isolated using a commercial kit, confirmed that MCF7 cells overexpressing Cx43 have Cx43 located at the plasma membrane **(Figure c).** Cx43 overexpression have been shown to induce proliferation arrest in different cancer cells. In order to determine if Cx43 overexpression in MCF7 cells affects cell proliferation, we performed a colony formation assay. Cells were plated at low density and cultured for 7 days. As shown in **Figure Id,** Cx43 overexpression significantly reduced cell proliferation and colony formation capacity in MCF7 cells after 7 days in culture.

### Example 2.2. Cx43 induces a senescent-like phenotype in Rb positive cancer cells

To understand the mechanism by which Cx43 overexpression reduces cell proliferation in Rb positive breast cancer cells, we decided to check if MCF7 cells overexpressing Cx43 acquire a senescent-like phenotype. As shown in **Figure 2a****,** Cx43 overexpression increases the levels of the senescent markers p21 and p53 accompanied by a reduction in the levels of Rb confirming a delay in the cell cycle compared to the vector control (EV). The increase in p21 levels was also confirmed using immunofluorescence **(****Figure 2b****).** Furthermore, Cx43 increases β-galactosidase activity, a key characteristic of senescent cells, in MCF7 confirming the induction of a senescent phenotype after Cx43 overexpression **(****Figure 2d****).** Besides, the secretion of SASP factors after Cx43 overexpression was tested using qPCR. The overexpression of Cx43 in MCF7 cells increases the secretion of different cytokines such as IL6, IL8 and MMP9 along with an increase in the gene expression of the senescent marker p21 **(****Figure 2c****).** Altogether, this data confirmed that Cx43 induces a senescent-like phenotype in MCF7 cells along with a reduction in cell proliferation and tumour growth.

### Example 2.3. Cx43 increases the efficacy of CDK4/6 inhibitors

In order to test if the treatment with the CDK4/6 inhibitor Palbociclib (Palbo) increases Cx43 levels in MCF7, we treated the cells with 250nM Palbo for 7 days and Cx43 protein levels were analysed by western-blot. As shown in **Figure 3a****,** Cx43 basal levels were slighted increased in MCF7 (EV) after Palbo treatment; however, this increase was higher if MCF7 cells overexpressing Cx43.

Based on the results obtained after Cx43 overexpression in MCF7 were we demonstrated that Cx43 reduces cell proliferation and, taking into account that CDK4/6 inhibitors induce a stable cell cycle arrest in different cancer cells, we decided to test if Cx43 overexpression also enhances the proliferation arrest and the senescent-like phenotype induced by CDK4/6 inhibitors. For that, cells expressing the vector control (EV) or Cx43 were treated for 7 days with Palbo and cell proliferation was analysed using a colony formation assay. As shown in **Figure 3b** the combination of Cx43 and Palbo treatment reduced the colony formation capacity of MCF7 cells demonstrating a synergic effect. To confirm if the reduction in proliferation was accompanied by an increase in senescence, we analyse the protein levels of the senescent marker p21 along with β-galactosidase activity. The data obtained demonstrated that the combination of Cx43 and Palbo increases the levels of p21 **(****Figure 3c****)** and β-galactosidase **(****Figure 3d****)** in MCF7 cells after 7 days treatment. To fully prove that the combination of Cx43 and CDK4/6 inhibitors could be a new therapeutic strategy to increase the efficacy of these inhibitors, we decided to test if the effect obtained with Palbo was maintained using Abemaciclib (Abema) and Ribociclib (Ribo). Colony formation assay demonstrated that Cx43 enhances the effect of Abema and Ribo by reducing cell proliferation in MCF7 cells **(****Figure 3 e-f****).** These data indicate that Cx43 increases the efficacy of CDK4/6 inhibitors (Palbo, Abema, Ribo) by decreasing proliferation and increasing cellular senescence.

### Example 2.4. The combination of Cx43 and CDK4/6 inhibitors enhances the effect of the senolytic drug Navitocax

Based on the data obtained where we demonstrated that Cx43 overexpression induced a senescent-like phenotype in Rb positive breast cancer cells, and that the combination with CDK4/6 inhibitors enhances the senescent phenotype, we tested whether the combination of Cx43 and CDK4/6 inhibitors would enhance the effect of the senolytic drug Navitoclax. As shown in **Figure 4a** the combination of Cx43, Palbociclib treatment and Navitoclax reduced cell proliferation compared to Cx43 overexpression and Palbociclib treatment together. To confirm that this reduction is due to the elimination of Cx43-senescent cells, breast cancer cells were treated with Palbociclib for 7 days and 24 hours with the senolytic drug Navitoclax. Cx43 was analysed by western-blot showing a reduction in the protein levels after the combination treatment due to the elimination of Cx43-tumour senescent cells (Cx43, Palbociclib, Navitoclax) **(****Figure 4b****).** This effect was accompanied by a reduction in the levels of different senescent markers such as β-galactosidase activity **(****Figure 4c****)** and the protein levels of p21, which increased under CDK4/6 inhibitors and Cx43 treatments and decreases in the presence of the senolytic drug due to the activation of cell death of the tumour senescent cells **(****Figure 4d****).** Furthermore, and to fully confirm the effect of Navitoclax in Cx43-senescent cells, proliferation was analysed using a colony formation assay after 14 days treatment **(****Figure 4e****),** demonstrating that the reduction in proliferation is higher in the presence of Cx43. In fact, the combination of Cx43, Palbociclib and Navitoclax, resulted in the best strategy to reduce the proliferation in breast cancer cells compared to Palbo and Navitoclax alone, probably due to the elimination of Cx43-positive senescent cells by the activation of programmed cell death by apoptosis. Interestingly, and in line with the results observed previously **(****Figure 4a****),** the treatment for 14 days revealed that the induction of senescence by Cx43 leads to an increase in the efficacy of Navitoclax (in presence of Cx43) **(****Figure 4e****),** indicating that Cx43 in long-term treatments further increases tumour cell senescence and can even result in a more effective treatment in comparison with CDK4/6 inhibitors treatment alone. Yet, the best therapeutic strategy resulted in the triple combination of CDK4/6 inhibitors, Cx43 and Navitoclax.

### Example 2.5. Cx43 increases the efficacy of CDK4/6 inhibitors in other tumours types

To explore if Cx43 increases the efficacy of CDK4/6 inhibitors in other tumour types, we took advantage of A375 melanoma cells and HT29 colon adenocarcinoma cell line. Cells (A375 and HT29) were transfected with a plasmid to overexpress Cx43. Rb and Cx43 protein levels were analysed by western-blot **(****Figure 5a** **and** **5c****).** As shown in **Figure 5b** and **Figure 5d****,** Cx43 overexpression increases the efficacy of Palbociclib, Abemaciclib and Ribociclib in melanoma and colon adenocarcinoma cell lines demonstrated by a reduction in proliferation after 7 days treatment.

### Example 2.6. sEVs containing high levels of Cx43 have anti-tumour activity and improve CDK4/6i efficacy

In order to further study the effect of Cx43, we have used small extracellular vesicles (sEVs) as a vehicle to delivery this protein to target cells. sEVs were isolated from HEK 293 cell line which contain very high levels of Cx43 **(****Figure 6a****).** To confirm that sEVs positives for Cx43 has an anti-proliferative effect, MCF7 cells were treated with HEK-293 derived-sEVs **(****Figure 6b****).**

## Claims

1. Connexin 43 for use in the treatment of cancer, wherein the cancer is **characterized by** the presence of functional retinoblastoma gene (RB).

2. Connexin 43 for use in the treatment of cancer, according to claim 1, wherein the cancer is further **characterized by** hyperactivity of CDK4 and CDK6.

3. Connexin 43 for use, according to any of the previous claims, in the treatment of cancer, before, after or simultaneously to a treatment with a CDK4/6 inhibitor.

4. Connexin 43 for use, according to any of the previous claims, in the treatment of cancer, before, after or simultaneously to a treatment with a CDK4/6 inhibitor selected from: Abemaciclib, Palbociclib and/or Ribociclib.

5. Connexin 43 for use, according to any of the previous claims, in the treatment of a cancer type selected from the list comprising: breast cancer, melanoma, lung cancer, colon, squamous cell lung cancer, pancreatic neuroendocrine tumour, oligodendroglioma and oligoastrocytoma.

6. Combination drug product comprising Connexin 43 and a CDK4/6 inhibitor.

7. Combination drug product, according to claim 6, wherein Connexin 43 can be selected between Connexin 43 protein or Connexin 43 mRNA.

8. Combination drug product, according to any of the claims 6 or 7, wherein the CDK4/6 inhibitor is selected from the group comprising: Abemaciclib, Palbociclib and/or Ribociclib.

9. Combination drug product, according to any of the claims 6 to 8, further comprising a senolytic agent, preferably navitoclax.

10. Pharmaceutical composition comprising the combination drug product of any of the claims 6 to 9 and, optionally, pharmaceutically acceptable excipients or carriers.

11. Pharmaceutical composition, according to claim 10, for use in the treatment of cancer wherein the cancer is positive for the retinoblastoma protein.

12. Pharmaceutical composition for use, according to claim 10, wherein the cancer is further **characterized by** hyperactivity of CDK4 and CDK6.

13. Pharmaceutical composition for use, according to claim 11 or 12, wherein Connexin 43 is administered by using a delivery vehicle.

14. Pharmaceutical composition for use, according to any of the claims 11 to 13, wherein the delivery vehicle is a nanoparticle, a vesicle, an extracellular vesicle, an expression vector or a molecule of DNA or RNA.

15. Pharmaceutical composition for use, according to any of the claims 11 to 14, wherein the expression vector encodes Connexin 43 mRNA which in turn is translated into Connexin 43 protein.
